# EUROPEAN PATENT APPLICATION

(11) **EP 3 677 679 A1**
(43) Date of publication of application: **08.07.2020**
(21) Application number: 17936076.3
(22) Date of filing: 26.12.2017
(51) Int. Cl.: C12N 15/11, A61K 48/00, A61P 3/06, A61P 9/10

(54) **SIRNA MOLECULE INHIBITING PCSK9 GENE EXPRESSION AND APPLICATION THEREOF**

(71) Applicant: Guangzhou Ribobio Co., Ltd., Guangzhou 510663 (CN)
(72) Inventor: ZHANG, Biliang, Guangzhou Development District Guangzhou, Guangdong 510663 (CN); YANG, Xiuqun, Guangzhou Development District Guangzhou, Guangdong 510663 (CN); WANG, Wei, Guangzhou Development District Guangzhou, Guangdong 510663 (CN)
(74) Representative: Biggi, Cristina
(86) International application number: PCT/CN2017/118526
(87) International publication number: WO 2019/126990

(57) **Abstract**

Provided are a small interfering RNA (siRNA) molecule which inhibits the expression of the PCSK9 gene and a pharmaceutical composition thereof, as well as a method for reducing the expression level of the PCSK9 gene by using the siRNA molecule or the pharmaceutical composition thereof. The siRNA molecules may be used to treat and/or prevent PCSK9 gene-mediated diseases.

SEQUENCE LISTING

<110>  Guangzhou RiboBio Co., Ltd.

<120>  SiRNA molecule inhibiting the expression of the PCSK9 gene and
       use thereof

<130>  IEC170136PCT

<160>  3

<170>  PatentIn version 3.5

<210>  1
<211>  21
<212>  RNA
<213>  Artificial Sequence

<400>  1
gacgaugccu gccucuacuu u                                                21


<210>  2
<211>  19
<212>  RNA
<213>  Artificial Sequence

<400>  2
gacgaugccu gccucuacu                                                   19


<210>  3
<211>  21
<212>  RNA
<213>  Artificial Sequence

<400>  3
aguagaggca ggcaucgucc c                                                21

## Description

### Technical Field

The present disclosure relates to the field of biomedicine. In particular, the present disclosure relates to a small interfering RNA (siRNA) molecule which inhibits the expression of the PCSK9 gene by an RNA interference technique and use thereof.

### Background

RNA interference (RNAi) refers to a phenomenon that is highly conserved during evolution, induced by double-stranded RNA, and that homologous mRNA is efficiently and specifically degraded. RNAi is widely found in natural species. Andrew Fire and Craig Mello et al., firstly discovered RNAi phenomenon in *Caenorhabditis elegans, C. elegans* in 1998. Tuschl and Phil Sharp etc. demonstrated in 2001 that RNAi is also present in mammals. After that, a series of progress has been made on the mechanism, gene function and clinical application of RNAi. RNAi plays a critical role in a variety of body protective mechanisms such as defense against viral infection, prevention of transposon jumping (Hutvagner et al., 2001; Elbashir et al., 2001; Zamore, 2001). The products developed based on RNAi mechanism are promising drug candidates.

Small interfering RNA (siRNA) can play an important role in RNA interference. Elbashir etc. found that siRNAs inhibit silencing of specific genes in mammalian cells in 2001. Studies have shown that siRNAs specifically binds to and degrades target mRNAs with complementary sequences. Longer double-stranded RNA are cleaved into shorter RNA by Dicer enzyme. An siRNA molecule consists of two strands, wherein one of the two strands that binds to a target mRNA is referred to as an antisense strand or a leader strand, and the other strand is referred to as a sense strand or a guest strand. It was found that siRNA synthesized in vitro could also exert RNA interference effect after entering cells, and effectively reduce the immune response induced by long RNAs. Thus, siRNA is a major tool for RNAi.

Proprotein convertase subtilisin/kexin type 9 (PCSK9) is a member of the subtilisin serine protease family that is involved in the regulation of low density lipoprotein receptor (LDLR) protein levels. The liver is the major site for expression of PCSK9. Other important sites for expression of PCSK9 include the pancreas, kidney, and intestine. LDLR may prevent atherosclerosis and hypercholesterolemia by removing low density lipoprotein (LDL) from the blood. Overexpression studies have shown that PCSK9 can control the level of LDLR. Meanwhile, it was found that: following knockout of the PCSK9 gene in mice, blood cholesterol levels are reduced and showed increased sensitivity to statins in lowering blood cholesterol. The above studies show that inhibitors of PCSK9 may be beneficial for the reduction of LDL-C (low density lipoprotein-cholesterol) concentration in blood, as well as for the treatment of PCSK9 mediated diseases.

Inhibitors targeting PCSK9 have been reported, as well as their use in the treatment of lipid disorders, but there is still a need to develop other inhibitors against this target for better efficacy, specificity, stability, targeting or tolerance.

### Summary

The disclosure provides an siRNA molecule for inhibiting the expression of a PCSK9 gene, a kit and a pharmaceutical composition thereof, and a method and use of the molecule, the kit or the pharmaceutical composition in inhibiting or reducing expression of the PCSK9 gene or treating diseases or symptoms mediated by the PCSK9 gene.

In particular, the present application relates to the present disclosure described below.
[1] An siRNA molecule for inhibiting the expression of a PCSK9 gene, containing a sense strand and an antisense strand complementary to form a double strand,
   wherein the sense and/or antisense strand contains or consists of 15-27 nucleotides and the antisense strand is complementary to at least 15, 16, 17, 18, 19, 20, 21, 22, 23, 24 or 25 consecutive nucleotides of SEQ ID NO: 1;
   and,
   wherein at least one nucleotide in the siRNA molecule is modified.
[2] The siRNA molecule of item [1], wherein the sense strand of the siRNA molecule contains the nucleotide sequence of SEQ ID NO: 1 or a nucleotide sequence of SEQ ID NO: 2; and the antisense strand of the siRNA molecule contains a nucleotide sequence of SEQ ID NO: 3.
[3] The siRNA molecule of item [1] or [2], wherein the modification contains a locked nucleic acid (LNA), unlocked nucleic acid (UNA), 2'-methoxyethyl, 2'-O-alkyl, 2'-O-methyl, 2'-O-allyl, 2'-C-allyl, 2'-fluoro, 2'-deoxy, 2'-hydroxy, phosphate backbone, DNA, fluorescent probe, ligand modification or combination thereof, preferably 2'-O-methyl, DNA, 2'-fluoro, thio-modified phosphate backbone or combination thereof.
[4] The siRNA molecule of item [3], wherein the sense and antisense strands are selected from the following combinations: strands 1 and 2; strands 3 and 5; strands 3 and 6; strands 3 and 7; strands 3 and 8; strands 9 and 4; strands 9 and 5; strands 9 and 6; strands 9 and 7; and strands 9 and 8.
[5] The siRNA molecule of item [3], wherein the ligand modification is performed at the 3' end, 5' end, or the middle of the sequence of the siRNA molecule;
   wherein the ligand moiety is Xm, wherein X is the same or different ligand selected from cholesterol, biotin, vitamins, galactose derivatives or analogs, lactose derivatives or analogs, N-acetylgalactosamine derivatives or analogs, N-acetylglucosamine derivatives or analogs, and any combination thereof, m is the number of ligands, preferably m = any integer from 1 to 5, more preferably, m = any integer from 2 to 4, most preferably m is 3.
[6] The siRNA molecule of item [5], wherein X has structure Z: 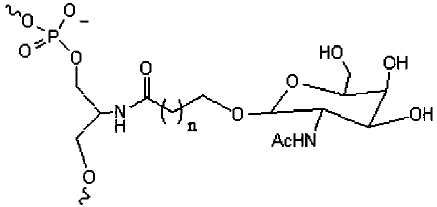 Z,
   wherein, the n value of the CH₂ groups in structure Z is independently selected from 1-15, preferably, the n value in structure Z is 3 or 8;
   more preferably, when m is 2, 3 or 4, the ligand moiety is (Z)₂, (Z)₃ or (Z)₄, respectively; most preferably each n value in (Z)₂, (Z)₃ or (Z)₄ is equal.
[7] The siRNA molecule of item [4], further containing a ligand and/or a fluorescent modification, wherein the ligand modification moiety is Xm, wherein X is the same or different ligand selected from cholesterol, biotin, vitamins, galactose derivatives or analogs, lactose derivatives or analogs, N-acetylgalactosamine derivatives or analogs, N-acetylglucosamine derivatives or analogs, and any combination thereof, and m is the number of ligands, preferably m = any integer from 1 to 5, more preferably m = any integer from 2 to 4, most preferably m is 3.
[8] The siRNA molecule of item [7], wherein the sense and antisense strands are selected from the following combinations: strands 13 and 8; strands 17 and 8; strands 19 and 8; strands 20 and 8; strands 13 and 10; strands 17 and 10; strands 19 and 10; strands 20 and 10; strands 14 and 10; strands 18 and 10; strands 21 and 10; and strands 22 and 10.
[9] The siRNA molecule of item [7], having structures of A:GACGAUGCCUGCCUCUACU-(X)m; fAmGfUfAfG(s)dA(s)dG(s)dGfCfAfG(s)dG(s)dC(s)dAfUfCmGfUfC(s)mC(s)mC; or B:mGmAmCfGfAfUmGmCmCfUfGfCfCmUfCfUmAmCmU-(X)m; fAmGfUfAfG(s)dA(s)dG(s)dGfCfAfG(s)dG(s)dC(s)dAfUfCmGfUfC(s)mC(s)mC;
   wherein X has structure Z: 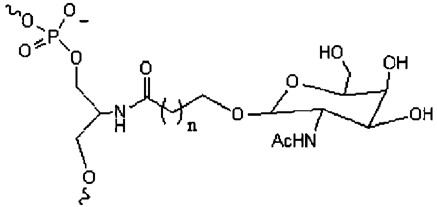 Z,
   the m value is 2 or 3 or 4;
   independently, the n value of the CH₂ groups in structure Z is selected from 1-15;
   preferably, when m is 2, 3 or 4, the ligand moiety is (Z)₂, (Z)₃ or (Z)₄, respectively, and each n value in (Z)₂, (Z)₃ or (Z)₄ is equal.
[10] The siRNA molecule of item [9], wherein n is 3 or 8.
[11] The siRNA molecule of any one of items [1]-[10] for inhibiting the expression of the PCSK9 gene in humans and monkeys.
[12] A pharmaceutical composition containing an siRNA molecule of any one of the items [1]-[11] and pharmaceutically acceptable other components.
[13] A method for inhibiting or reducing the expression level of the PCSK9 gene in a cell in vivo or in vitro, comprising introducing into the cell the siRNA molecule of any one of items [1]-[11], or the pharmaceutical composition of item [12], such that the expression level of the PCSK9 gene is inhibited or reduced by at least 95%, at least 90%, at least 85%, at least 80%, at least 75%, at least 70%, at least 60%, at least 50%, at least 40%, at least 30%, at least 20%, at least 10%, or at least 5%.
[14] Use of the siRNA molecule of any one of items [1]-[11] or the pharmaceutical composition of item [12] for the manufacture of a medicament for inhibiting or reducing the expression level of the PCSK9 gene in a cell in vivo or in vitro or for the manufacture of a medicament for treating diseases or symptoms mediated by the PCSK9 gene in a subject.
[15] The use of item [14], wherein the diseases or symptoms mediated by the PCSK9 gene contains a cardiovascular disease or a neoplastic disease, preferably the cardiovascular disease is selected from hyperlipidemia, hypercholesterolemia, non-familial hypercholesterolemia, polygenic hypercholesterolemia, familial hypercholesterolemia, homozygous familial hypercholesterolemia, or heterozygous familial hypercholesterolemia and the neoplastic disease is selected from melanoma, hepatocellular carcinoma, and metastatic liver cancer.
[16] A kit containing an siRNA molecule of any one of the items [1]-[11].
[17] A method for treating diseases or symptoms mediated by the PCSK9 gene in a subject, containing administering to the subject the siRNA molecule of any one of items [1]-[11] or the pharmaceutical composition of item [12].
[18] The use of item [17], wherein the diseases or symptoms mediated by the PCSK9 gene contains a cardiovascular disease or a neoplastic disease, preferably the cardiovascular disease is selected from hyperlipidemia, hypercholesterolemia, non-familial hypercholesterolemia, polygenic hypercholesterolemia, familial hypercholesterolemia, homozygous familial hypercholesterolemia, or heterozygous familial hypercholesterolemia.
[19] Use of the siRNA molecule of any one of items [1]-[11] or a pharmaceutical composition of item [12] in the manufacture of a medicament for inhibiting or reducing the expression level of the PCSK9 gene in a cell in vivo or in vitro.
[20] Use of the siRNA molecule of any one of items [1]-[11] or the pharmaceutical composition of item [12] for inhibiting or reducing the expression level of the PCSK9 gene in a cell in vivo or in vitro.

Embodiments of the present disclosure will now be described in detail with reference to the accompanying drawings and examples, however, it will be understood by those skilled in the art that the following drawings and examples are merely illustrative of the present disclosure and are not intended to limit the scope of the present disclosure. Various objects and advantageous aspects of the present disclosure will become apparent to those skilled in the art from the accompanying drawings and the following detailed description of the preferred embodiments.

### Brief Description of the Drawings

Figures 1A-C show binding curves of GaINAc-siRNAs of the present disclosure to receptors.
Figure 2 shows ex vivo organ imaging after euthanasia 6 hours after the administration.
Figure 3 shows ex vivo organ imaging after euthanasia 6 hours after the administration.

### Sequence information

Information about the sequences to which the present disclosure relates is provided in the following table:

**The basic sequences (unmodified) are as follows:**

| Sequence No (SEQ ID NO:) | |
|---|---|
| 1 | GACGAUGCCUGCCUCUACUUU(sense strand) |
| 2 | GACGAUGCCUGCCUCUACU(sense strand) |
| 3 | AGUAGAGGCAGGCAUCGUCCC(antisense strand) |

**Each strand in each siRNA molecule involved in the present application and the composition thereof are as follows:**

| Strand No | Strand composition |
|---|---|
| 1 | GACGAUGCCUGCCUCUACUmU(s)mU |
| 2 | AGUAGAGGCAGGCAUCGUCmC(s)mC |
| 3 | GACGAUGCCUGCCUCUACU |
| 4 | AGUAGAGGCAGGCAUCGUCCC |
| 5 | fA(s)dGfU(s)dA(s)dGdAmGGfCAGGfCAfUfCGfUfC(s)mC(s)mC |
| 6 | fA(s)dGfU(s)dA(s)dG(s)dA(s)dGfGfCmAmGmGfCfAfUfCmGfUfC(s)mC(s)mC |
| 7 | fA(s)dGfU(s)dA(s)dG(s)dAmGmGmCmAfGfGfCmAfUmCfGfUfC(s)mC(s)mC |
| 8 | fAmGfUfAfG(s)dA(s)dG(s)dGfCfAfG(s)dG(s)dC(s)dAfUfCmGfUfC(s)mC(s)m C |
| 9 | mGmAmCfGfAfUmGmCmCfUfGfCfCmUfCfUmAmCmU |
| 10 | Cy5-fAmGfUfAfG(s)dA(s)dG(s)dGfCfAfG(s)dG(s)dC(s)dAfUfCmGfUfC(s)mC(s)mC |
| 11 | GACGAUGCCUGCCUCUACU-L |
| 12 | GACGAUGCCUGCCUCUACU-LL |
| 13 | GACGAUGCCUGCCUCUACU-LLL |
| 14 | GACGAUGCCUGCCUCUACU-LLLL |
| 15 | GACGAUGCCUGCCUCUACU-S |
| 16 | GACGAUGCCUGCCUCUACU-SS |
| 17 | GACGAUGCCUGCCUCUACU-SSS |
| 18 | GACGAUGCCUGCCUCUACU-SSSS |
| 19 | mGmAmCfGfAfUmGmCmCfUfGfCfCmUfCfUmAmCmU-LLL |
| 20 | mGmAmCfGfAfUmGmCmCfUfGfCfCmUfCfUmAmCmU-SSS |
| 21 | mGmAmCfGfAfUmGmCmCfUfGfCfCmUfCfUmAmCmU-LLLL |
| 22 | mGmAmCfGfAfUmGmCmCfUfGfCfCmUfCfUmAmCmU-SSSS |
| 23 | GACGAUGCCUGCCUCUACUUU |

| | |
|---|---|
| Note: "G", "C", "A", "T" and "U" each generally represent a nucleotide that contains guanine, cytosine, adenine, thymine and uracil as a base, respectively. Modifications: d=DNA; m = 2'-O-methyl; f = 2'-fluoro; (s) = PS backbone (i.e. thio-modified phosphate backbone); L= L-type ligand; S = S-type ligand; Cy5 = fluorescently labeled Cy5. | |

### Detailed Description of the Embodiments

Embodiments of the present disclosure are described in more detail below.

The present disclosure provides an siRNA molecule for inhibiting the expression of the PCSK9 gene, which contains a sense strand and an antisense strand complementary to form a double strand, wherein the sense strand and/or the antisense strand contains or consists of 15-27 nucleotides, and the antisense strand is complementary to at least 15, 16, 17, 18, 19, 20, 21, 22, 23, 24, or 25 contiguous nucleotides of SEQ ID NO:1, and wherein at least one nucleotide in the siRNA molecule is modified.

As used herein, the term "antisense strand" refers to a strand of siRNA including a region that is completely or substantially complementary to a target sequence. As used herein, the term "complementary region" refers to a region of the antisense strand that is completely or substantially complementary to the target mRNA sequence. Where the complementary region is not fully complementary to the target sequence, the mismatch may be in an internal or terminal region of the molecule. Typically, the most tolerated mismatch is in the terminal region, e.g., within 5, 4, 3, 2, or 1 nucleotide of the 5' and/or 3' end. The portion of the antisense strand that is most sensitive to mismatches is referred to as the "seed region". For example, in siRNA containing a 19 nt strand, the 19th position (from 5' to 3') can tolerate some mismatch.

As used herein, the term "complementary" refers to the ability of a first polynucleotide to hybridize to a second polynucleotide under certain conditions, such as stringent conditions. For example, stringent conditions may include 400 mM NaCl, 40 mM PIPES pH 6.4, 1 mM EDTA at 50°C or 70°C for 12-16 hours.

As used herein, the term "sense strand" refers to a strand of siRNA that includes a region that is substantially complementary to a region of the term antisense strand as defined herein.

The antisense and sense strands of the siRNA may be of the same or different lengths, as described herein and as known in the art.

The siRNA molecules promote sequence-specific degradation of PCSK9 mRNA by RNAi to achieve inhibition the expression of the PCSK gene or reduction of the expression level of the PCSK9 gene, e.g., by 95%, 90%, 85%, 80%, 75%, 70%, 60%, 50%, 40%, 30%, 20%, 10%, or 5%.

At least 70%, 80%, 90%, 95%, or 100% of the nucleotides of each strand of the siRNA molecule are ribonucleotides, but may also contains one or more non-ribonucleotides, such as deoxyribonucleotides.

In some embodiments, at least one nucleotide in the siRNA molecule of the present disclosure is modified. Although many modifications can be attempted to improve the performance of siRNA, these attempts are often difficult to address both mediating RNA interference and improving stability in serum (e.g., increased resistance to nucleases and/or prolonged duration). The modified siRNA of the present disclosure has high stability while maintaining high inhibitory activity.

Modifications suitable for the present disclosure may be selected from the group containing: locked nucleic acid (LNA), unlocked nucleic acid (UNA), 2'-methoxyethyl, 2'-O-alkyl, 2'-O-methyl, 2'-O-allyl, 2'-C-allyl, 2'-fluoro, 2'-deoxy, 2'-hydroxy, phosphate backbones, fluorescent probes, ligand modifications, or combinations thereof. Preferred modifications are 2'-O-alkyl groups such as 2'-O-methyl, DNA, 2'-fluoro, thio-modified phosphate backbones, and combinations thereof.

In particular embodiments of the present disclosure, preferred modifications include: (1) for the antisense strand: the first nucleotide at the 5' end is modified by 2'-fluorine, the second nucleotide is modified by 2'-O-methyl, the middle region is a repeat structure of fNfNfN(s)dN(s)dN(s)dNfNfNfN(s)dN(s)dN(s)dN, and the 3' end has a 5'(s)mN(s)mN 3' pendant structure; the nucleotides between the pendant structure and the intermediate region are modified with 2'-O-methyl or 2'-fluoro, with up to 2 consecutive 2'-fluoro or 2'-O-methyl modifications. (2) the sense strand is not modified, or all nucleotides of the sense strand are modified: the nucleotide with 12nt at the 5' end is modified by mNmNmNfNfNfNmNmNmNfNfNfN, and the nucleotide with 3nt at the 3' end is modified by 2'-O-methyl; the intermediate nucleotides are modified with 2'-O-methyl or 2'-fluoro, with up to 2 consecutive 2'-fluoro or 2'-O-methyl modifications. In some embodiments, the siRNA molecules provided herein are selected from RBP9-005-P1G1, RBP9-005-P2G2, RBP9-005-P2G3, RBP9-005-P2G4, RBP9-005-P2G5, RBP9-005-P2G6, RBP9-005-P3G2, RBP9-005-P3G3, RBP9-005-P3G4, RBP9-005-P3G5, and RBP9-005-P3G6 shown in Table 5.

In some embodiments, the chemically modified siRNA molecule is preferably RBP9-005-P2G6 or RBP9-005-P3G6.

In some embodiments, ligand modification may be performed at the 3' end, 5' end, or in the middle of the sequence of the siRNA molecule. In some embodiments, the ligand may be a moiety that is taken up by the host cell. Ligands suitable for the present disclosure include cholesterol, biotin, vitamins, galactose derivatives or analogs, lactose derivatives or analogs, N-acetylgalactosamine derivatives or analogs, N-acetylglucosamine derivatives or analogs, or combinations thereof. Preferably, the ligand modification is an N-acetylgalactosamine derivative or analogue modification.

The ligand modification may improve cellular uptake, intracellular targeting, half-life, or drug metabolism or kinetics of siRNA molecules. In some embodiments, the ligand-modified siRNA has enhanced affinity or cellular uptake for a selected target (e.g., a particular tissue type, cell type, organelle, etc.), preferably a hepatocyte, as compared to the siRNA without ligand modification. Preferred ligands do not interfere with the activity of the siRNA.

In some embodiments, the siRNAs of the present disclosure may contains one or more, preferably 1-5, 2-4 or 3, ligand modifications, preferably N-acetylgalactosamine derivatives/analogs.

In some embodiments, the N-acetylgalactosamine derivative ligand modification moiety has structure Z:

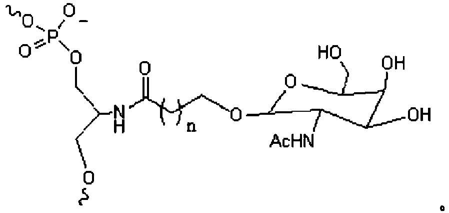

In some embodiments, the ligand-modified siRNA molecule is selected from any of P2G6-03L, P2G6-03S, P3G6-03L, and P3G6-03S shown in Table 8, and P2G6-13L, P2G6-13S, P3G6-13L, and P3G6-13S shown in Table 10.

In some embodiments, the ligand-modified siRNA molecular has the following structures:
GACGAUGCCUGCCUCUACU-ZZZ;
fAmGfUfAfG(s)dA(s)dG(s)dGfCfAfG(s)dG(s)dC(s)dAfUfCmGfUfC(s)mC(s)mC; or
mGmAmCfGfAfUmGmCmCfUfGfCfCmUfCfUmAmCmU-ZZZ;
fAmGfUfAfG(s)dA(s)dG(s)dGfCfAfG(s)dG(s)dC(s)dAfUfCmGfUfC(s)mC(s)mC;
wherein structure ZZZ is connected with the nucleotide at 3'-end of the siRNA sense strand through a phosphodiester bond, and each Z is connected with the adjacent Z through a phosphodiester bond; wherein structure ZZZ linked to siRNA is as follows: 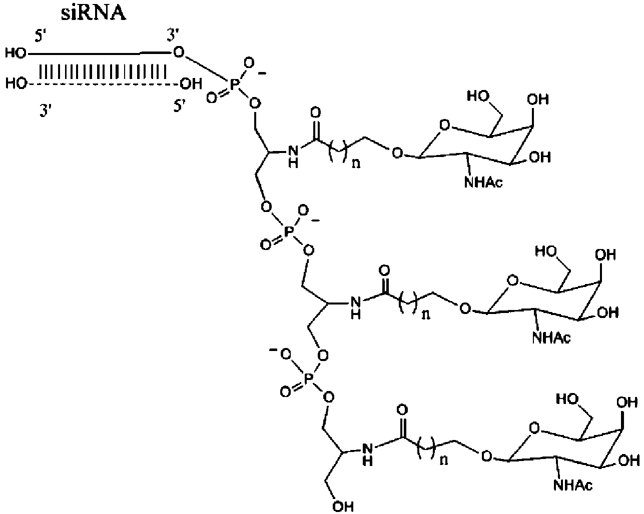 the n value of the CH₂ group in each Z is independently selected from 1-15; preferably, the n values in structure ZZZ are equal.

In one embodiment, the n values in structure ZZZ are all 3. When the n value is 3, Z can be represented by L.

In one embodiment, the n values in structure ZZZ are all 8. When the n value is 8, Z can be represented by S. The siRNA molecules of the present disclosure may have from 0% to 100%, such as 5%, 10%, 15%, 20%, 25%, 30%, 35%, 40%, 45%, 50%, 55%, 60%, 65%, 70%, 75%, 80%, 85%, 90%, or more than 95% modified nucleotides in each strand. The modification may be in the pendant region or in the double-stranded region. The modifications of the present disclosure can be used to improve in vitro or in vivo characteristics of siRNA molecules, such as stability, biodistribution, inhibitory activity, etc. Modifications of the present disclosure may be used in combination.

Each strand of the siRNA molecules of the present disclosure has a pendant end or a blunt end. The 5' and/or 3' ends of either or both of the antisense or sense strands may be pendant ends. The pendant end may have 1-8 pendencies, preferably 2, 3, 4, 5, or 6 pendencies, wherein the pendency is selected from any of U, A, G, C, T, and dT.

In some embodiments, the siRNA molecules of the present disclosure inhibit PCSK9 gene expression in humans or monkeys.

In some embodiments, the present disclosure also provides a vector for inhibiting the expression of the PCSK9 gene in a cell, which expresses at least one strand of an siRNA molecule of the present disclosure. As used herein, the term "vector" refers to a nucleic acid molecule capable of amplifying or expressing another nucleic acid to which it is linked.

In some embodiments, the present disclosure also provides a kit containing an siRNA molecule or vector of the present disclosure.

In some embodiments, the present disclosure also provides a pharmaceutical composition containing an siRNA molecule or vector of the present disclosure and a pharmaceutically acceptable other component. In one embodiment, the compositions of the present disclosure comprise a pharmacologically effective amount of the siRNA molecule or vector of the present disclosure and pharmaceutically acceptable other components. As used herein, the term "effective amount" refers to an amount of the siRNA molecule effective to produce the desired pharmacological therapeutic effect. "Other components" include water, saline, dextrose, buffers (e.g., PBS), excipients, diluents, disintegrants, binders, lubricants, sweeteners, flavoring agents, preservatives, or any combination thereof.

In some embodiments, the present disclosure also provides a method for inhibiting or reducing the expression level of the PCSK9 gene in a cell in vivo or in vitro, containing introducing into the cell an siRNA molecule, vector, kit or pharmaceutical composition of the present disclosure such that the expression level of the PCSK9 gene is inhibited or reduced by at least 95%, at least 90%, at least 85%, at least 80%, at least 70%, at least 60%, at least 50%, at least 40%, at least 30%, at least 20%, at least 10%, at least 5%. As used herein, the term "introducing" refers to facilitating uptake or absorption into a cell, which may occur through non-assisted diffusion or active cellular processes, or through an ancillary agent or device. When introduced into cells in vivo, siRNA can be injected into the target tissue or administered systemically. Introduction to cells in vitro may be by methods known in the art, such as electroporation.

The cell is preferably a mammalian cell expressing PCSK9, for example a primate cell, such as a human cell. Preferably, the PCSK9 gene is expressed at a high level in the target cell. More preferably, the cells are derived from brain, salivary gland, heart, spleen, lung, liver, kidney, intestinal tract or tumor. Even more preferably, the cell is a hepatoma cell or a cervical cancer cell. Still even more preferably, the cells are selected from HepG2 and HeLa cells.

In an in vitro method, the final cellular concentration of the siRNA molecule is 0.1-1000 nM, preferably 10-500 nM, 25-300 nM, or 50-100 nM.

Detection of the level of the target gene, target RNA, or target protein can be used to predict or assess the activity, efficacy, or therapeutic outcome of the siRNA. Detection of target gene, target RNA or target protein levels can be carried out using methods known in the art.

In some embodiments, the present disclosure provides an in vivo method containing alleviating or treating diseases or symptoms mediated by a PCSK gene in a subject, the diseases or symptoms containing cardiovascular disease, dyslipidemia; cardiovascular diseases may include atherosclerotic cardiovascular diseases, and dyslipidemia may include elevated cholesterol and/or triglyceride levels, elevated low density lipoprotein cholesterol, or elevated apolipoprotein B (ApoB) in the serum. The diseases or symptoms is preferably hyperlipidemia, hypercholesterolemia, non-familial hypercholesterolemia, polygenic hypercholesterolemia, familial hypercholesterolemia, homozygous familial hypercholesterolemia, or heterozygous familial hypercholesterolemia. The subject may be a mammal, preferably a human.

As used herein, the term "hypercholesterolemia" refers to a condition characterized by elevated serum cholesterol. The term "hyperlipidemia" refers to a condition characterized by elevated serum lipids. The term "non-familial hypercholesterolemia" refers to a condition characterized by increased cholesterol that is not caused by a single genetic mutation. The term "polygenic hypercholesterolemia" refers to a condition characterized by increased cholesterol resulting from the effects of a variety of genetic factors. The term "familial hypercholesterolemia (FH)" refers to an autosomal dominant metabolic disorder characterized by mutations in the LDL-receptor (LDL-R), significantly increased LDL-C, and premature onset of atherosclerosis. The term "homozygous familial hypercholesterolemia" or "HoFH" refers to a condition characterized by mutations in the maternal and paternal LDL-R genes. The term "heterozygous familial hypercholesterolemia" or "HoFH" refers to a condition characterized by mutations in the maternal or paternal LDL-R gene.

PCSK gene-mediated diseases or symptoms may result from overexpression of the PCSK9 gene, overproduction of the PCSK9 protein, and may be modulated by down-regulation of PCSK9 gene expression. As used herein, the term "treatment" refers to alleviation, relieving, or cure of the PCSK9 gene-mediated diseases or symptoms, such as a decrease in blood lipid levels, including serum LDL, LDL-C levels. In some embodiments, the levels or concentrations of serum LDL, serum LDL-C are reduced by 5%, 10%, 15%, 20%, 25%, 30%, 35%, 40%, 50%, 60%, 70%, 80%, or 90%.

In an in vivo method, the pharmaceutical composition may be administered by any suitable means, such as parenteral administration, including intramuscular, intravenous, arterial, intraperitoneal, or subcutaneous injection. Modes of administration include, but are not limited to, single administration or multiple administrations. The dosage administered may range from 0.1 mg/kg to 100 mg/kg, 0.5 mg/kg to 50 mg/kg, 2.5 mg/kg to 20 mg/kg, 5 mg/kg to 15 mg/kg, preferably 3 mg/kg, 10 mg/kg, 33 mg/kg, more preferably 10 mg/kg, most preferably 33 mg/kg.

In another aspect, the present disclosure also provides a method of combination therapy containing co-administration of one or more siRNAs of the present disclosure or pharmaceutical compositions containing the same with one or more other therapeutic agents or in combination with other therapeutic methods. Other therapeutic agents may include agents known to prevent, alleviate or treat lipid disorders such as hypercholesterolemia, atherosclerosis or dyslipidemia, such as cholesterol absorption inhibitors, lipid lowering agents, analgesics, anti-inflammatory agents, antineoplastic agents, and the like. Other methods of treatment include radiation therapy, immunotherapy, hormonal therapy, surgical therapy, and the like.

The siRNA molecule provided by the present disclosure has multiple beneficial effects: 1, the modified siRNA molecule has high stability and high inhibition activity; 2, the siRNA molecule modified by the ligand not only keeps high inhibition activity and stability, but also has good liver targeting property and capability of promoting endocytosis, can reduce the influence on other tissues or organs and the use amount of the siRNA molecule, thereby achieving the purposes of reducing toxicity and reducing cost; and 3, the ligand-modified siRNA molecules can enter target cells and target tissues without a transfection reagent, reducing the negative influence of the transfection reagent, such as cell or tissue toxicity. Thus, the ligand-modified siRNA molecules of the present disclosure provide the potential for targeted therapy.

### Abbreviations, acronyms, and numbers

"G", "C", "A", "T" and "U" each generally represents a nucleotide with guanine, cytosine, adenine, thymine and uracil as bases, respectively.
REL(Relative expression level): relative expression level of mRNA.
GalNAc: N-acetylgalactosamine.
Modifications: N=RNA; dN=DNA; mN = 2'-O-methyl modification; fN = 2'-fluoro modification; (s) = PS backbone (i.e. 5'-thio modified phosphate backbone).
Abbreviations: RBP9-005-P3G6 may be referred to simply as P3G6, and so on.
Numbers: the first position following the broken line of P3G6 indicates whether there is a fluorescent label (0 indicates "none", 1 indicates "yes"), the second position indicates the number of ligands, and the third position indicates the type of ligands, e.g., P3G6-13L indicates that P3G6 is fluorescently labeled, and 3 L ligands are modified; and so on.

### EXAMPLES

### Example 1 Activity screening of PCSK9-siRNA

### SiRNA Design

Multiple of pairs of PCSK9-siRNAs were designed by selecting different sites according to human pcsk9mRNA sequences, all designed single siRNAs could target all transcripts of target genes (such as table 1), and the multiple pairs of siRNAs had the lowest homology with all other non-target gene sequences through alignment by a sequence similarity software. The sequence design method is referred to Elbashir et al.2002; Paddison et al.2002; Reynoldset al.2004; Ui-Tei et al.2004, and so on.

**Table 1 Target genes**

| Target gene | Species | Gene ID | NM_ID |
|---|---|---|---|
| PCSK9 | Homo sapiens (human) | 255738 | NM_174936.3 |

### Synthesis of siRNA

The oligonucleotides of the 2'-hydroxyl-containing ribonucleotides of the present disclosure were all completed according to the theoretical yield of 1 µmol synthesis specifications. In anhydrous acetonitrile, the weighed 1 µmol of standard universal solid support CPG or 3'-cholesterol modified CPG (purchased from Chemgenes), 2'-O-TBDMS protecting RNA phosphoramidite monomer, DNA monomer, 2'-methoxy monomer and 2'-fluoro monomer (purchased from Sigma Aldrich) were dissolved to a concentration of 0.2 M. For phosphorothioate backbone modified oligonucleotides, 0.2 M PADS solution was used as the thionating reagent. A solution of 5-ethylthio-IH-tetrazole (purchased from Chemgenes) in acetonitrile as the activator (0.25 M), a 0.02 M solution of iodine in pyridine/water as the oxidant, and a 3% solution of trichloroacetic acid in dichloromethane as the deprotecting reagent were placed at the designated position of the reagent corresponding to the ABI 394 DNA/RNA automated synthesizer. A synthesis procedure was set up and the designated oligonucleotide base sequence was entered. After error checking, cycle oligonucleotide synthesis was started. The coupling time for each step was 6 minutes and the coupling time for the galactose ligand for the L and S monomers was 10-20 minutes. After automatic circulation, oligonucleotide solid-phase synthesis was completed. The CPG was blown dry with dry nitrogen, transferred to a 5 ml EP tube, added with 2 ml ammonia/ethanol solution (3/1) and heated at 55°C for 16-18 hours. Centrifugation was carried out at 10,000 rpm for 10 min, the supernatant was taken and concentrated aqueous ammonia/ethanol was pumped off to dryness to give a white gummy solid. The solid was dissolved in 200 µL of 1 M TBAF in THF and shaken at room temperature for 20 h. Then 0.5 ml of 1 M Tris-HCI buffer (pH 7.4) was added, shaken for 15 minutes at room temperature and placed on a centrifugal pump to a volume of 1/2 of the original volume to remove THF. The resulting solution was extracted twice with 0.5 ml of chloroform, 1 ml of 0.1 M TEAA loading solution was added, the mixed solution was poured into a solid phase extraction column to remove excess salt in the solution. The resulting oligonucleotide concentration was determined by micro-UV spectrophotometer (KO5500). The mass spectrometric analysis was performed on an Oligo HTCS LC-MS system (Novatia) system. Nucleic acid molecular weights were calculated by normalization using Promass software after primary scanning.

Only deoxyribonucleotide-or 2'-methoxy-or 2'-fluoro-or LNA-or 2'-MOE-containing modified oligonucleotides according to the present disclosure were carried out according to a theoretical yield of 1 µmol synthetic specifications. In anhydrous acetonitrile, the weighed 1 µmol of standard universal solid support CPG or 3'-cholesterol modified CPG (purchased from Chemgenes), DNA monomer, 2'-methoxy monomer and 2'-fluoro monomer (purchased from Sigma Aldrich) were dissolved to a concentration of 0.2 M. For phosphorothioate backbone modified oligonucleotides, 0.2 M PADS solution was used as the thionating reagent. A solution of 5-ethylthio-IH-tetrazole (purchased from Chemgenes) in acetonitrile as the activator (0.25 M), a 0.02 M solution of iodine in pyridine/water as the oxidant, and a 3% solution of trichloroacetic acid in dichloromethane as the deprotecting reagent were prepared and placed at the designated position of the reagent corresponding to the ABI 394 DNA/RNA automated synthesizer. A synthesis procedure was set up and the designated oligonucleotide base sequence was entered. After error checking, cycle oligonucleotide synthesis was started. The coupling time for each step was 6 minutes and the coupling time for the galactose ligand for the monomers was 6-10 minutes. After automatic circulation, oligonucleotide solid-phase synthesis was completed. The CPG was blown dry with dry nitrogen, transferred to a 5 ml EP tube, added with 2 ml ammonia solution and heated at 55°C for 16-18 hours. Centrifugation was carried out at 10,000 rpm for 10 min the supernatant was taken and concentrated aqueous ammonia/ethanol was pumped off to dryness to give a white or yellow gummy solid. Followed by adding 1 ml of 0.1 M TEAA loading solution, the mixed solution was poured onto a solid phase extraction column to remove excess salt from the solution. The resulting oligonucleotide concentration was determined by micro-UV spectrophotometer (KO5500). The mass spectrometric analysis was performed on an Oligo HTCS LC-MS system (Novatia) system. Nucleic acid molecular weights were calculated by normalization using Promass software after primary scanning.

### Transfection of different cells with PCSK9-siRNA

All cells were from ATCC or other publicly available sources; other reagents are commercially available.

**Table 2 Cell names and species**

| Cell names | Huh7 | HePG2 | MHCC97H | HeLa |
|---|---|---|---|---|
| Cell species | Liver cancer cells | Liver cancer cells | Liver cancer cells | Cervical cancer cells |

Cells were cultured in DMEM medium containing 10% fetal bovine serum in a 5% CO₂, 37°C constant temperature incubator. Transfection with the transfection reagents was performed when the cells were in logarithmic growth phase and in a good condition (70% confluence). The cell concentration was adjusted to 1 x 10⁶/mL, and mL of cell solution and 5 µL of riboFect transfection reagent were added to each well of a 6-well plate. After standing for 5 min at room temperature, 5 µL of 100 nM siRNA were added and incubated at 37°C and 5% CO₂ for 48 h.

For each cell plating, the following control groups were set up in addition to the experimental group: NC as negative control (irrelevant siRNA), Mock as transfection reagent control group, and untreated control group (UT group, no siRNA added). The test group and the control group were repeated 3 times.

### Real-time quantitative PCR analysis of target mRNA levels

1, Cells were lysed 48 h after transfection and total RNA was extracted by Trizol method.
2, A reverse transcription kit Reverse Transcription mix was used for reverse transcription (Guangzhou RiboBio Co., Ltd.).
3, Fluorescent quantitative PCR:
Using β-actin gene as internal reference gene, real-time fluorescence quantitative PCR was performed using SYBR Premix (2x), a real-time PCR kit. PCR reactions were performed using a CFX96 fluorescent quantitative PCR instrument of the Bio-Rad, USA. The primers used were as follows:

**Table 3 Primers**

| | |
|---|---|
| PCSK9-qPCR-F(5'-3') | AAGCCAAGCCTCTTCTTACTTCA |
| PCSK9-qPCR-R(5'-3') | CCTGGGTGATAACGGAAAAAG |

4, Data Analysis

At the end of the PCR reaction, 9 replicates of one sample (3 transfection replicates per sample, 3 qPCR replicates) had a Ct error of ± 0.5. CFX 2.1 was used for relative quantitative analysis. Table 4 is a mean value of the target gene expression level relative to group NC (the mRNA relative expression level of group NC is 1). The results of real-time quantitative PCR detection of preferred siRNAs are shown in the following table.

**Table 4 Real-time quantitative PCR detection results**

| Names | Sequence (5'-3') | Strand No | HePG2 (relative expression levels) | HeLa (relative expression levels) |
|---|---|---|---|---|
| NC | UGAAGAGCCUGAUCAAAUAdTdT | | 1 | 1 |
| | UAUUUGAUCAGGCUCUUCAdTdT | | | |
| RBP9-001 | CAGAGACUGAUCCACUUCUmU(s)m U | | 0.75 | 0.35 |
| | AGAAGUGGAUCAGUCUCUGmC(s)m C | | | |
| RBP9-003 | CCUGGAGUUUAUUCGGAAAmU(s)m U | | 0.71 | 0.08 |
| | UUUCCGAAUAAACUCCAGGmC(s)m C | | | |
| RBP9-004 | CUGCUGAGCUGCUCCAGUUmU(s)m U | | 0.73 | 0.26 |
| | AACUGGAGCAGCUCAGCAGmC(s)m U | | | |
| RBP9-005 | GACGAUGCCUGCCUCUACUmU(s)m U | 1 | 0.55 | 0.10 |
| | AGUAGAGGCAGGCAUCGUCmC(s)m C | 2 | | |
| RBP9-007 | GCAGCCUGGUGGAGGUGUAmU(s)m U | | 0.78 | 0.18 |
| | UACACCUCCACCAGGCUGCmC(s)m | | | |
| | U | | | |
| RBP9-013 | CACGAGGUCAGCCCAACCAmU(s)m U | | 1.15 | 0.28 |
| | UGGUUGGGCUGACCUCGUGmG(s) mC | | | |
| RBP9-022 | GCCUGGAGUUUAUUCGGAAmU(s)m U | | 0.76 | 0.08 |
| | UUCCGAAUAAACUCCAGGCmC(s)m U | | | |

PCSK9 siRNA screenings in HepG2 and HeLa reveal a highly active siRNA molecule, i.e. RBP9-005, in HeLa and HePG2 cells.

### Example 2 PCSK9-siRNA optimization

Different modifications and optimizations were performed on RBP9-005. The steps of synthesis, transfection, and quantitative PCR were the same as in Example 1. The transfected cells were HeLa and HePG2 cells, and the results are shown in Table 5 (REL-H: relative expression level of PCSK9 mRNA in HeLa cells; REL-2: relative expression level of PCSK9 mRNA in HePG2 cells). Table 5 is a mean value of the target gene expression level relative to group NC (the mRNA relative expression level of group NC is 1). Wherein, NC was an irrelevant siRNA negative control group, Mock was a transfection reagent control group, and UT was an untreated cell control group.

**Table 5 RBP9-005 Optimization**

| Samples | Modified sequences (5'-3') | Strand No | REL-H | SD | REL-2 | SD |
|---|---|---|---|---|---|---|
| NC | UGAAGAGCCUGAUCAAAUA dTdT | | 1 | 0.05 | 1 | 0.06 |
| | UAUUUGAUCAGGCUCUUCAd TdT | | | | | |
| Mock | / | | 1.03 | 0.07 | 1.05 | 0.03 |
| | / | | | | | |
| UT | / | | 0.97 | 0.04 | 0.95 | 0.06 |
| | / | | | | | |
| RBP9-005-P1G1 | GACGAUGCCUGCCUCUACUm U(s)mU | 1 | 0.10 | 0.04 | 0.53 | 0.04 |
| | AGUAGAGGCAGGCAUCGUCm C(s)mC | 2 | | | | |
| RBP9-005-P2G2 | GACGAUGCCUGCCUCUACU | 3 | 0.10 | 0.03 | 0.51 | 0.05 |
| | AGUAGAGGCAGGCAUCGUCC C | 4 | | | | |
| RBP9-005-P2G3 | GACGAUGCCUGCCUCUACU | 3 | 0.09 | 0.04 | 0.46 | 0.03 |
| | fA(s)dGfU(s)dA(s)dGdAmGGfCA GGfCA fUfCGfUfC (s)mC(s)mC | 5 | | | | |
| RBP9-005-P2G4 | GACGAUGCCUGCCUCUACU | 3 | 0.16 | 0.07 | 0.50 | 0.03 |
| | fA(s)dGfU(s)dA(s)dG(s)dA(s)dGf GfCmAmGmGfCfAfUfCmGfUfC( s)mC(s)mC | 6 | | | | |
| RBP9-005-P2G5 | GACGAUGCCUGCCUCUACU | 3 | 0.15 | 0.06 | 0.55 | 0.03 |
| | fA(s)dGfU(s)dA(s)dG(s)dAmGm GmCmAfGfGfCmAfUmCfGfUfC( s)mC(s)mC | 7 | | | | |
| RBP9-005-P2G6 | GACGAUGCCUGCCUCUACU | 3 | 0.06 | 0.01 | 0.46 | 0.05 |
| | fAmGfUfAfG(s)dA(s)dG(s)dGfCf AfG(s)dG(s)dC(s)dAfUfCmGfUfC (s)mC(s)mC | 8 | | | | |
| RBP9-005-P3G2 | mGmAmCfGfAfUmGmCmCfUfG fCfCmUfCfUmAmCmU | 9 | 0.13 | 0.05 | 0.47 | 0.05 |
| | AGUAGAGGCAGGCAUCGUCC C | 4 | | | | |
| RBP9-005-P3G3 | mGmAmCfGfAfUmGmCmCfUfG fCfCmUfCfUmAmCmU | 9 | 0.13 | 0.03 | 0.52 | 0.02 |
| | fA(s)dGfU(s)dA(s)dGdAmGGfCA GGfCAfUfCGfUfC(s)mC(s)mC | 5 | | | | |
| RBP9-005-P3G4 | mGmAmCfGfAfUmGmCmCfUfG fCfCmUfCfUmAmCmU | 9 | 0.17 | 0.04 | 0.50 | 0.04 |
| | fA(s)dGfU(s)dA(s)dG(s)dA(s)dGf GfCmAmGmGfCfAfUfCmGfUfC( s)mC(s)mC | 6 | | | | |
| RBP9-005-P3G5 | mGmAmCfGfAfUmGmCmCfUfG fCfCmUfCfUmAmCmU | 9 | 0.18 | 0.04 | 0.52 | 0.03 |
| | fA(s)dGfU(s)dA(s)dG(s)dAmGm GmCmAfGfGfCmAfUmCfGfUfC( s)mC(s)mC | 7 | | | | |
| RBP9-005-P3G6 | mGmAmCfGfAfUmGmCmCfUfG fCfCmUfCfUmAmCmU | 9 | 0.09 | 0.03 | 0.41 | 0.03 |
| | fAmGfUfAfG(s)dA(s)dG(s)dGfCf AfG(s)dG(s)dC(s)dAfUfCmGfUfC (s)mC(s)mC | 8 | | | | |

The results showed that the inhibitory activities of chemically modified RBP9-005-P2G6 and RBP9-005-P3G6 are higher in HeLa and HePG2 cells.

### Example 3 Targeting Assay of GalNAc-siRNA Cells

### First. Preparation of ligand-modified siRNAs

### 1, Synthesis of Galactose Modified Monomer L

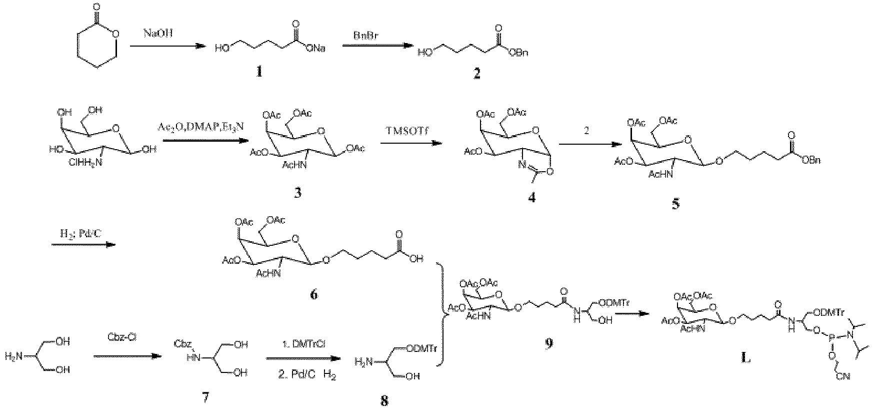

### 1) Synthesis of compound 1

In a 1 L round bottom flask, δ-valerolactone (100 g, 1 mol), sodium hydroxide (40 g, 1 mol) and 400 mL of deionized water were mixed, reacted for 6 hours at 70°C, and monitored by TCL until the reaction was completed. The reaction was spin-dried, added with 200 ml of toluene, followed by spin drying to get 140 g of a white solid.

### 2) Synthesis of compound 2

In a 1 L round bottom flask, compound 1 (140 g, 1 mol), anhydrous acetone 500 mL, benzyl bromide (205.2 g, 1.2 mol), and catalyst tetrabutylammonium bromide (16.2 g, 0.05 mol) were added and refluxed under heating. The reaction was monitored by TLC and was complete after 24 h. After the reaction liquid was cooled to room temperature, acetone was removed under reduced pressure. The residue was dissolved in 500 mL of ethyl acetate and washed successively with 200 mL of saturated sodium bisulfate, 200 mL of saturated sodium bicarbonate and 200 mL of saturated brine. The organic phase was dried over anhydrous sodium sulfate, concentrated and passed through a silica gel column (petroleum ether: ethyl acetate V: V = 1: 1) to isolate 175 g of a clear oily liquid with a yield of 84%.

### 3) Synthesis of compound 3

In a 1 L round bottom flask, D-galactose hydrochloride (100 g, 0.46 mol) and 450 mL of anhydrous pyridine were added, and 325 mL of acetic anhydride, triethylamine (64.5 mL, 0.46 mol) and DMAP (2 g, 0.016 mol) were slowly added under an ice bath. After overnight reaction at room temperature, a large amount of solid was precipitated. Suction filtration was carried out to give a filter cake, which was rinsed with 200 mL of 0.5 N HCI solution to get 162.5 g of a white solid with a yield of 90%. ¹H NMR(400MHz,DMSO-d6)δ: 7.88(d,J=9.2Hz,1H), 5.63(d,J=8.8Hz,1H), 5.26(d,J= 3.1Hz, 1H), 5.05(d,J= 11.3,3.3Hz,1H), 4.36(m,4H), 2.11(s,3H), 2.03(s,3H), 1.98(s,3H), 1.90(s,3H), 1.78(s,3H).

### 4) Synthesis of compound 4

In a 250 mL round bottom flask, compound 3 (10 g, 25.7 mmol) and 100 mL of anhydrous dichloromethane was added. After stirring for 10 minutes trimethylsilyl trifluoromethanesulfonate (7 mL, 38.7 mmol) was added. The reaction was then allowed to proceed overnight at room temperature. The reaction solution was slowly poured into an aqueous solution (200 mL) of sodium bicarbonate (7 g, 79.5 mmol) and stirred for 0.5 hours. The organic phase was separated and dried over anhydrous sodium sulfate. After concentration under reduced pressure, 7.78 g of light yellow colloid was obtained with a yield of 92%.

### 5) Synthesis of Compound 5

In a 100 mL round bottom flask, compound 4 (5 g, 15.2 mmol) and compound 2 (3.8 g, 18.25 mmol) were dissolved in 50 mL of anhydrous1, 2-dichloroethane, stirred for 10 minutes, and added with trimethylsilyl trifluoromethanesulfonate (0.55 mL, 3 mmol). Reaction was allowed to proceed overnight. The reaction liquid was extracted with dichloromethane. The obtained organic phase was washed twice with 50 mL of saturated sodium bicarbonate, dried over anhydrous sodium sulfate, then concentrated under reduced pressure and passed through a silica gel column (petroleum ether: ethyl acetate V: V = 3: 2) to isolate 6.94 g of a clear oily liquid with a yield of 85%. ¹HNMR(400MHz,DMSO-d6)δ: 7.69(d,J=9.3Hz,1H), 7.33-7.16(m,5H), 5.28(d,J =5.3Hz,1 H), 4.95(s,2H), 4.93(q,J=4.2Hz,1H), 4.40(d,J=8.6Hz,1 H), 4.00-3.86(m,3H), 3.73-3.56(m,2H), 3.36-3.21(m,1H), 2.53(t,J=8.2Hz,2H), 2.11(s,3H), 1.89(s,3H), 1.83(s,3H), 1.65(s,3H), 1.59-1.36(m,4H).MS(ESI), m/z:560.2([M+Na] ⁺).

### 6) Synthesis of compound 6

In a 50 mL round bottom flask, compound 5 (3.3 g, 6.1 mmol), Pd/C (0.33 g, 10%) were dissolved in 5 mL of methanol and 20 mL of ethyl acetate, then introduced with a hydrogen balloon and reacted overnight at room temperature. The reaction liquid was filtered through diatomite, and the diatomite was then rinsed with methanol. The filtrate was concentrated under reduced pressure and spin-dried to get 2.8 g of a white solid with a yield of 95.5%. ¹HNMR(400MHz,DMSO-d6)δ: 11.98(s,1H), 7.79(d,J = 8.9Hz,1H), 5.20(s,1H), 5.0-4.95(q,J = 4.2Hz,1H), 4.51-4.46(d,J = 7.2Hz,1H), 4.15-3.97(m,3H), 3.89-3.79(m,1H), 3.80-3.69(m,1H), 3.46-3.36(m,1H), 2.22-2.14(t,J = 7.2Hz,2H), 2.15(s,3H), 2.00(s,3H), 1.95(s,3H), 1.87(s,3H), 1.59-1.42(m,4H).MS(ESI), m/z:470.5 ([M+Na]⁺).

### 7) Synthesis of compound 7

With serine as raw material, compound 7, a white solid, was synthesized referred to Choi J Y et al with a yield of 89%. MS(ESI), m/z:248.2([M+Na]⁺).

### (8) Synthesis of compound 8

Compound 8 was synthesized from compound 2 with reference to US 2011/0077389 A1. A white solid was obtained with a yield of 56%. ¹HNMR(400MHz,DMSO-d6)δ: 7.41-7.37(d,J = 7.2Hz,2H), 7.33-7.28(t,J = 6.9Hz,2H), 7.27-7.19(m,5H), 6.91-6.86(d,J=8.2Hz,4H), 5.16(s,2H), 4.63-4.58(m,1H), 4.05-3.97(m,1H), 3.74(s,6H), 3.04-2.99(m,2H), 2.95-2.90(m,2H).MS(ESI), m/z:416.3([M+Na]⁺).

### (9) Synthesis of compound 9

In a 250 mL round bottom flask, compound 6 (10 g, 22.35 mmol), 1-ethyl-(3-dimethylaminopropyl) carbonyldiimine hydrochloride (EDC. HCL) (5.14 g, 26.82 mmol), N-hydroxysuccinimide (2.83 g, 24.59 mmol), and dichloromethane 100 mL were added. After reacting under stirring at room temperature for 0.5 h, compound 8 (8.79 g, 22.35 mmol) was added. The reaction was monitored by TLC and was complete after 4 h. The reaction liquid was washed successively with 50 mL of saturated sodium bicarbonate and 50 mL of saturated brine, the organic phase was dried over anhydrous sodium sulfate, then concentrated, and passed through a silica gel column (dichloromethane: methanol V: V = 20: 1) to isolate 15.8 g of a white solid with a yield of 86%. MS(ESI), m/z:845.2([M+Na]⁺).

### (10) Synthesis of compound monomer L

In a 250 mL two-necked flask, compound 1 (5 g, 6.08 mmol) under nitrogen protection, 100 mL anhydrous acetonitrile, and bis (diisopropylamino)(2-cyanoethoxy) phosphine (3.66 g, 12.16 mmol) were added, and a solution of ethylthiotetrazole in acetonitrile (2.5 M) (1.22 mL, 3.04 mmol) was slowly added dropwise with stirring. Reaction was carried out for 0.5 h. The reaction was monitored by TLC and was complete after 0.5 h. The acetonitrile was removed by concentration under reduced pressure. 100 ML of dichloromethane was added to dissolve the concentrate, followed by washing with 100 mL of saturated brine. The organic phase was dried over anhydrous sodium sulfate, concentrated and passed through a silica gel column (petroleum ether: ethyl acetate V: V = 1:3) to isolate 5.16 g of a white solid with a yield of 83%. ¹H NMR(400MHz,DMSO-d6)δ: 7.84-7.79(d,J=8.9Hz,1H), 7.65-7.60(d,J= 8.9Hz,1H), 7.41-7.37(d,J=7.2Hz,2H), 7.33-7.28(t, J=6.9Hz,2H), 7.27-7.19(m,5H), 6.91-6.86(d,J = 8.2Hz,4H), 5.20(s,1H), 5.0-4.95(q,J = 4.2Hz,1H), 4.51-4.46(d,J = 7.2Hz,1H), 4.15-3.97(m,3H), 4.05-3.96(m,1H), 3.84-3.80(m,2H), 3.89-3.79(m,1H), 3.74(s,6H), 3.71-3.69(m,1H), 3.46-3.36(m,1H), 3.04-2.99(m,2H), 2.95-2.90(m,2H), 2.88-2.84(m,2H), 2.59-2.54(m,2H), 2.22-2.14(t,J=7.2Hz,2H),2.15(s,3H),2.00(s,3H), 1.95(s,3H), 1.87(s,3H), 1,77(s,12H), 1.59-1.42(m,4H).MS(ESI), m/z:1045.5([M+Na]⁺).

### 2, synthesis of galactose monomer S

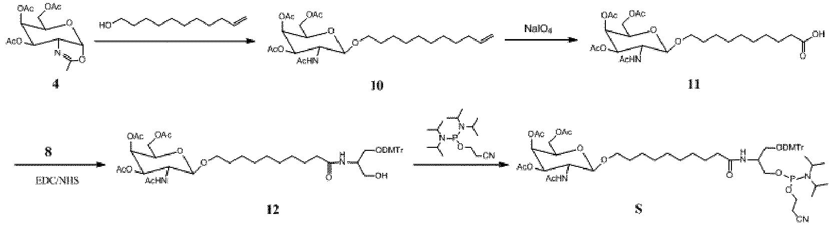

### (1) Synthesis of compound 10

In a 100 mL round bottom flask, compound 4 (5 g, 15.2 mmol) and 10-undecenol (3.1 g, 18.24 mmol) dissolved in 50 ml of anhydrous dichloromethane were added. After stirring for 10 minutes trimethylsilyl trifluoromethanesulfonate (0.55 mL, 3.0mmol) was added. Reaction was allowed to proceed overnight. The reaction liquid was extracted with dichloromethane. The resulting organic phase was washed twice with 50 mL of saturated sodium bicarbonate, then dried over anhydrous sodium sulfate, concentrated under reduced pressure and passed through a silica gel column (petroleum ether: ethyl acetate V: V = 3: 2) to isolate 6.59 g of a white solid with a yield of 87%. ¹HNMR(400MHz,DMSO-d6)δ : 7.82(d,J = 3.3Hz,1H), 5.86-5.73(m,1H), 5.22(s,1H), 5.02-4.9(m,3H), 4.5-4.98(s,J = 3.5Hz,1H), 4.08-3.99(m,3H), 3.9-3.88(m,1H), 3.73-3.65(m,1H), 3.48-3.38(m,1H), 2.12(s,3H), 2.05-2.01(m,2H), 2.00(s,3H), 1.88(s,3H), 1.66(s,3H), 1.5-1.4(m,2H), 1.39-1.3(m,2H), 1.29-1.19(m,10H).MS(ESI), m/z:522.4([M+Na]⁺).

### (2) Synthesis of compound 11

In a 100 mL round bottom flask, compound 10 (4g, 8.02mmol), 50 mL of dichloromethane, 50 mL of acetonitrile, and 70 mL of deionized water were added. NalO₄ (6.86 g, 32.1 mmol) was added portion-wise. The reaction was carried out at room temperature for 48 h. The completion of the reaction was monitored by TCL. The reaction solution was added with 100 ML of deionized water, and then extracted three times with dichloromethane (50 mL x 3). The organic phases were combined, dried over anhydrous sodium sulfate, concentrated under reduced pressure and spin-dried to get 4.1 g of a pale brown gummy product with a yield of 99%. ¹HNMR(400MHz,DMSO-d6)δ: 11.99(s,1H), 7.82(d,J=3.3Hz,1H), 5.22(s,1H), 5.02-4.9(m,1H), 4.5-4.98(s,J = 3.5Hz,1H), 4.08-3.99(m,3H), 3.9-3.88(m,1H), 3.73-3.65(m,1H), 3.48-3.38(m,1H), 2.12(s,3H), 2.05-2.01(m,2H), 2.00(s,3H), 1.88(s,3H), 1.66(s,3H), 1.5-1.4(m,2H), 1.39-1.3(m,2H), 1.29-1.19(m,10H).MS(ESI), m/z:540.26([M+Na]⁺).

### (3)Synthesis of compound 12

Referring to the synthesis of compound 1, a white solid was obtained with a yield of 85.6%. MS (ESI), m/z: 915.5 ([M + Na]+).

### (4) Synthesis of Compound S

Referring to the synthesis of compound L, a white solid was obtained with a yield of 82.1%.
¹HNMR(400MHz,DMSO-d6)δ : 7.82-7.78(d,J = 7.3Hz,1H), 7.69-7.63(d,J = 7.3Hz,1H), 7.41-7.37(d,J = 7.2Hz,2H), 7.33-7.28(t,J = 6.9Hz,2H), 7.27-7.19(m,5H), 6.91-6.86(d,J = 8.2Hz,4H), 5.22(s,1H), 5.02-4.9(m,1H), 4.5-4.98(s,J = 3.5Hz,1H), 4.08-3.99(m,3H), 4.05-3.97(m,1H), 3.9-3.88(m,1H), 3.84-3.80(m,2H), 3.74(s,6H), 3.73-3.65(m,1H), 3.48-3.38(m,1H), 3.04-2.99(m,2H), 2.95-2.90(m,2H), 2.88-2.84(m,2H), 2.61-2.55(m,2H), 2.12(s,3H), 2.05-2.01(m,2H), 2.00(s,3H), 1.88(s,3H), 1.77(s,12H), 1.66(s,3H), 1.5-1.4(m,2H), 1.39-1.3(m,2H), 1.29-1.19(m,10H).MS(ESI), m/z:1115.2([M+Na]⁺).

### 3, Synthesis of GalNac-siRNA

See Example 1.

### Second. Isolation of primary mouse hepatocytes

Mice were anesthetized and the skin and muscle layers were excised to expose the liver. A perfusion catheter was inserted into the portal vein, and the inferior vena cava was cut open to prepare for liver perfusion. Perfusion solutions Solution I (Hank' s, 0.5 mM EGTA, pH 8) and Solution II (low glucose DMEM, 100 U/mL Type IV, pH 7.4) were preheated at 40°C. Solution I of 37°C was perfused into the liver via the portal vein cannula at a flow rate of 7 mL/min for 5 min until the liver turned off-white. Solution II of 37°C was perfused into the liver at a flow rate of 7 mL/min for 7 min. After perfusion was complete, the liver was removed and placed in Solution III (10% FBS-low glucose DMEM, 4°C) to stop digestion, then the liver capsule was cut by the forceps, and the hepatocytes was released by gently shaking. The hepatocytes were filtered through a 70 µm cell filter, centrifuged at 50 g for 2 min with the supernatant discarded. The cells were resuspended with Solution IV (40% percoll low glucose DMEM, 4°C), then centrifuged at 100 g for 2 min with the supernatant discarded. Cells were added with 2% FBS-low glucose DMEM and resuspended in for use. Cell viability was identified by Trypan blue staining.

### Third, Determinations of GalNAc binding curves and Kd values

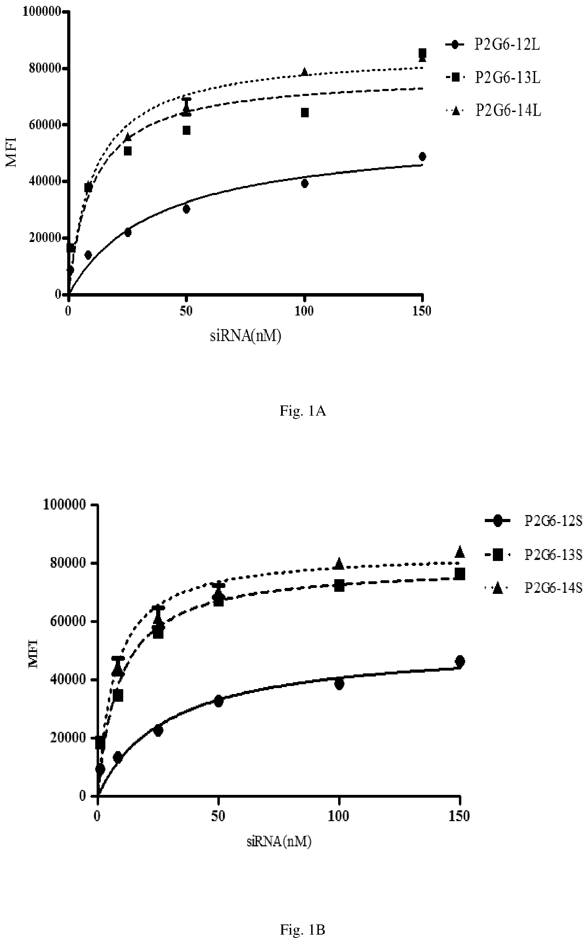

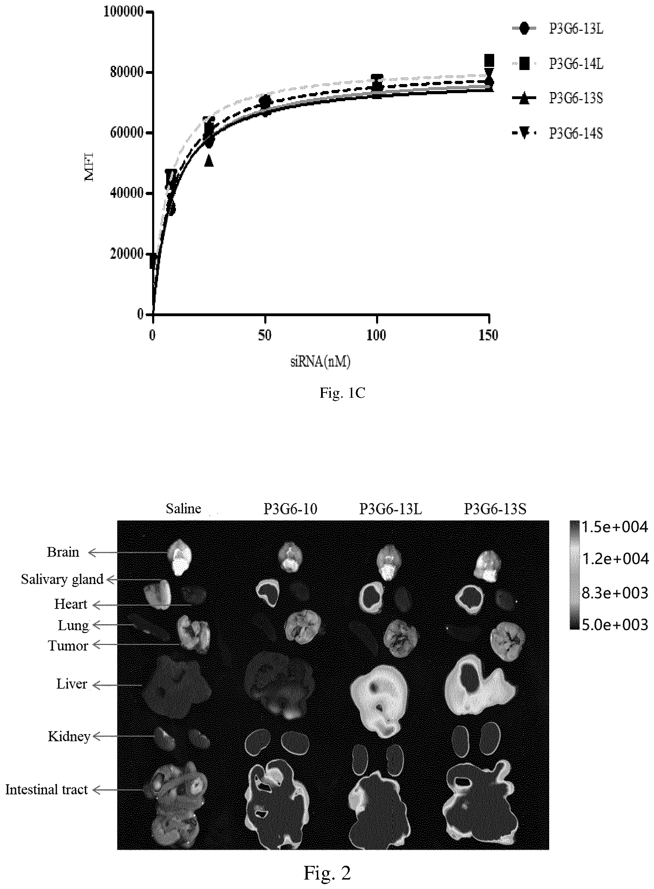

Freshly isolated mouse primary hepatocytes were plated in 96-well plates at 2 x 10⁴ cells/well, 100 µl/well. GalNAc-siRNA was added to each well, respectively. Each GalNAc-siRNA was set to a final concentration of 0.9 nM, 8.3 nM, 25 nM, 50 nM, 100 nM, 150 nM, respectively. The suspensions were incubated at 4°C for 2 h, and centrifuged at 50 g for 2 min with the supernatant discarded. The cells were resuspended in 10 µg/ml PI, stained for 10 min and centrifuged at 50 g for 2 min. Cells were washed with pre-cooled PBS and centrifuged at 50 g for 2 min with the supernatant discarded. The cells were resuspended in PBS. The mean fluorescence intensity MFI of living cells was measured by a flow cytometry, and nonlinear fitting and Kd value calculation were performed by the GraphPad Prism 5 software. The data in Tables 6A-C and Figures 1A-C show that ligand-modified GaINAc-siRNAs promote in vitro hepatocyte endocytosis/uptake without the addition of transfection reagents, achieving delivery of hepatocytes. Meanwhile, GaINAc-siRNAs with different GalNac structures show different endocytosis and receptor binding abilities. According to Bmax and Kd values, GalNAc-siRNA with structures 3S, 4S and 3L, 4S have better affinity to hepatocytes.

**Table 6 A. Kd Values and Bmax values for each experimental group**

| | P2G6-12L | P2G6-13L | P2G6-14L |
|---|---|---|---|
| Bmax | 57245 | 77943 | 85917 |
| Kd | 37.9 | 10.4 | 10.9 |

**Table 6 B. Kd Values and Bmax values for each experimental group**

| | P2G6-12S | P2G6-13S | P2G6-14S |
|---|---|---|---|
| Bmax | 52542 | 79308 | 83928 |
| Kd | 29.4 | 9.2 | 7.1 |

**Table 6C. Kd values and Bmax values for each experimental group**

| | P3G6-13L | P3G6-14L | P3G6-13S | P3G6-14S |
|---|---|---|---|---|
| Bmax | 80242 | 82685 | 78521 | 81661 |
| Kd | 9.6 | 6.8 | 8.9 | 8.8 |

**Table 7. GalNAc-siRNA Sequence structures for targeting assays**

| No | Structures (5'-3') | Strand No | Descriptions |
|---|---|---|---|
| P2G6-10 | GACGAUGCCUGCCUCUACU | 3 | Cy5+, Targeting- |
| | Cy5-fAmGfUfAfG(s)dA(s)dG(s)dGfCfAfG( s)dG(s)dC(s)dAfUfCmGfUfC(s)mC(s)mC | 10 | |
| P2G6-11L | GACGAUGCCUGCCUCUACU-L | 11 | Cy5+, 3' L |
| | Cy5-fAmGfUfAfG(s)dA(s)dG(s)dGfCfAfG( s)dG(s)dC(s)dAfUfCmGfUfC(s)mC(s)mC | 10 | |
| P2G6-12L | GACGAUGCCUGCCUCUACU-LL | 12 | Cy5+, 3'LL |
| | Cy5-fAmGfUfAfG(s)dA(s)dG(s)dGfCfAfG( s)dG(s)dC(s)dAfUfCmGfUfC(s)mC(s)mC | 10 | |
| P2G6-13L | GACGAUGCCUGCCUCUACU-LLL | 13 | Cy5+, 3'LLL |
| | Cy5-fAmGfUfAfG(s)dA(s)dG(s)dGfCfAfG( s)dG(s)dC(s)dAfUfCmGfUfC(s)mC(s)mC | 10 | |
| P2G6-14L | GACGAUGCCUGCCUCUACU-LLLL | 14 | Cy5+, 3' LLLL |
| | Cy5-fAmGfUfAfG(s)dA(s)dG(s)dGfCfAfG( s)dG(s)dC(s)dAfUfCmGfUfC(s)mC(s)mC | 10 | |
| P2G6-11S | GACGAUGCCUGCCUCUACU-S | 15 | Cy5+, 3'S |
| | Cy5-fAmGfUfAfG(s)dA(s)dG(s)dGfCfAfG( s)dG(s)dC(s)dAfUfCmGfUfC(s)mC(s)mC | 10 | |
| P2G6-12S | GACGAUGCCUGCCUCUACU-SS | 16 | Cy5+, 3'SS |
| | Cy5-fAmGfUfAfG(s)dA(s)dG(s)dGfCfAfG( s)dG(s)dC(s)dAfUfCmGfUfC(s)mC(s)mC | 10 | |
| P2G6-13S | GACGAUGCCUGCCUCUACU-SSS | 17 | Cy5+, 3'SSS |
| | Cy5-fAmGfUfAfG(s)dA(s)dG(s)dGfCfAfG( s)dG(s)dC(s)dAfUfCmGfUfC(s)mC(s)mC | 10 | |
| P2G6-14S | GACGAUGCCUGCCUCUACU-SSSS | 18 | Cy5+, 3' SSSS |
| | Cy5-fAmGfUfAfG(s)dA(s)dG(s)dGfCfAfG( s)dG(s)dC(s)dAfUfCmGfUfC(s)mC(s)mC | 10 | |
| P3G6-10 | mGmAmCfGfAfUmGmCmCfUfGfCfCmUf CfUmAmCmU | 9 | Cy5+, Targeting- |
| | Cy5-fAmGfUfAfG(s)dA(s)dG(s)dGfCfAfG( s)dG(s)dC(s)dAfUfCmGfUfC(s)mC(s)mC | 10 | |
| P3G6-13L | mGmAmCfGfAfUmGmCmCfUfGfCfCmUf CfUmAmCmU-LLL | 19 | Cy5+, 3'LLL |
| | Cy5-fAmGfUfAfG(s)dA(s)dG(s)dGfCfAfG( s)dG(s)dC(s)dAfUfCmGfUfC(s)mC(s)mC | 10 | |
| P3G6-13S | mGmAmCfGfAfUmGmCmCfUfGfCfCmUf CfUmAmCmU-SSS | 20 | Cy5+, 3'SSS |
| | Cy5-fAmGfUfAfG(s)dA(s)dG(s)dGfCfAfG( s)dG(s)dC(s)dAfUfCmGfUfC(s)mC(s)mC | 10 | |
| P3G6-14L | mGmAmCfGfAfUmGmCmCfUfGfCfCmUf CfUmAmCmU-LLLL | 21 | Cy5+, 3'LLLL |
| | Cy5-fAmGfUfAfG(s)dA(s)dG(s)dGfCfAfG( s)dG(s)dC(s)dAfUfCmGfUfC(s)mC(s)mC | 10 | |
| P3G6-14S | mGmAmCfGfAfUmGmCmCfUfGfCfCmUf CfUmAmCmU-SSSS | 22 | Cy5+, 3' SSSS |
| | Cy5-fAmGfUfAfG(s)dA(s)dG(s)dGfCfAfG( s)dG(s)dC(s)dAfUfCmGfUfC(s)mC(s)mC | 10 | |

Note: 1 following the broken line in P2G6-10 indicates that the modification is carried out through a fluorescent label, and 0 following the broken line indicates that the modification is not carried out through a targeting ligand; 1 following the broken line in P2G6-13L indicates that the modification is carried out through a fluorescent label, and 3L following the broken line indicates that the targeting ligand is modified into 3 L (LLL); other annotations and so on.

### Example 4 In vitro efficacy test of GaINAc-siRNAs

Referring to the procedure of Example 1, mRNA expression levels in HePG2 cells were measured. The final concentration of GalNAc-siRNA transfection was 50 nM. The results are shown in Table 8.

**Table 8 In vitro inhibitory activity of GaINAc-siRNAs**

| | HePG2 Cells (with transfection reagent) REL |
|---|---|
| P2G6 | 0.47 |
| P2G6-03L | 0.50 |
| P2G6-03S | 0.51 |
| P3G6 | 0.40 |
| P3G6-03L | 0.38 |
| P3G6-03S | 0.36 |

**Table 9. GalNAc-siRNA Sequence structures for efficacy test**

| No | Structures (5'-3') | Strand No |
|---|---|---|
| P2G6-03L | GACGAUGCCUGCCUCUACU-LLL | 13 |
| | fAmGfUfAfG(s)dA(s)dG(s)dGfCfAfG(s)dG(s)dC(s)d AfUfCmGfUfC(s)mC(s)mC | 8 |
| P2G6-03S | GACGAUGCCUGCCUCUACU-SSS | 17 |
| | fAmGfUfAfG(s)dA(s)dG(s)dGfCfAfG(s)dG(s)dC(s)d AfUfCmGfUfC(s)mC(s)mC | 8 |
| P3G6-03L | mUmCmAfUmAmGmGfCfCfUmGmGfAmGfUfUmU mAmU-LLL | 19 |
| | fAfU(s)dA(s)dA(s)dAfCfUfCfCAfGfGfCfCfUmAfUfGf A(s)mG(s)mG | 8 |
| P3G6-03S | mUmCmAfUmAmGmGfCfCfUmGmGfAmGfUfUmU mAmU-SSS | 20 |
| | fAfU(s)dA(s)dA(s)dAfCfUfCfCAfGfGfCfCfUmAfUfGf A(s)mG(s)mG | 8 |

Note: 0 following the broken line in P2G6-13L indicates that the modification is not carried out through a fluorescent label, and 3L following the broken line indicates that the targeting ligand is modified into 3 L (LLL); other annotations and so on.

### Example 5 In vivo Liver Targeting Assay

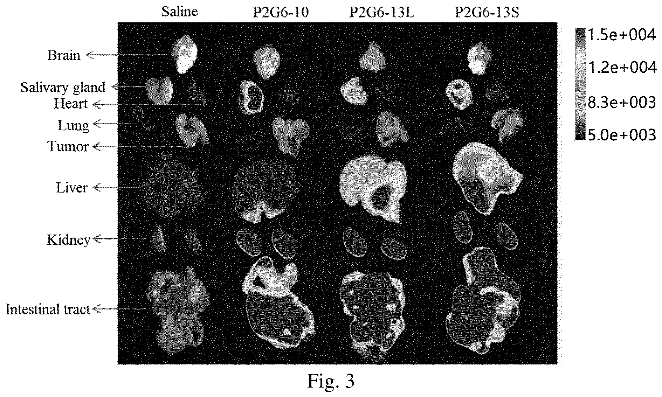

Twenty-four (24) Male, 6-7 week old SPF grade Balb/c-nu mice (purchased from Beijing Vital River Laboratory Animal Technology Co., Ltd.) were used for this study. The mice were randomly divided into 7 groups, including blank control group, group P2G6-10, group P3G6-10, group P2G6-13L, group P2G6-13S, group P3G6-13L and group P3G6-13S. The number of animals in each group was 2, 3, 3, 4, 4, 4 and 4 respectively. Mice were administered by caudal vein injection at a dose of approximately 10 mg/kg (see Table 10 for experimental design). All animals were subjected to in vivo imaging, including white light and X-ray imaging, before dosing, and 5 min, 30 min, 1 h, 2 h, 4 h, 6 h after dosing. After euthanasia 6 hours after the administration, the brain, salivary glands, heart, spleen, lung, liver, kidney, and intestinal tract were removed for imaging analysis of isolated organs (Figures 2 and 3).

**Table 10. Liver targeting experimental design**

| Sequence No | Groupings | Injection products | Dosing (mg/kg) | Dosing Volume (mL) |
|---|---|---|---|---|
| 1 | Blank control | Saline | 0 | 0.2 |
| 2 | NC1 | P2G6-10 | 10 | 0.2 |
| 3 | NC2 | P3G6-10 | 10 | 0.2 |
| 4 | Positive group | P2G6-13L | 10 | 0.2 |
| 5 | Positive group | P2G6-13S | 10 | 0.2 |
| 6 | Positive group | P3G6-13L | 10 | 0.2 |
| 7 | Positive group | P3G6-13S | 10 | 0.2 |

Ex vivo imaging analyses were performed and the results are shown in Tables 11 and 12. The results showed that the fluorescence intensities of the livers of the groups P2G6-13L, P2G6-13S, P3G6-13L, and P3G6-13S were significantly higher than that of the negative control group (NC) 6 hours after administration (see fluorescence intensity ratio results in Table 12), indicating that P2G6-13L, P2G6-13S, P3G6-13L, and P3G6-13S have significantly higher targeting to the liver.

**Table 11. Statistics of ex vivo organ fluorescence intensity values after background subtraction x10⁸ ps/mm²)**

| Groupings | Animal No | Salivary gland | Liver | Kidney | Intestinal tract |
|---|---|---|---|---|---|
| P2G6-10 | Average values | 9728 | 3556 | 45038 | 17644 |
| | SD | 1023 | 570 | 4725 | 819 |
| P2G6-13L | Average values | 9247 | 8280 | 33232 | 17246 |
| | SD | 1257 | 2078 | 559 | 1911 |
| P2G6-13S | Average values | 7278 | 12957 | 39642 | 20212 |
| | SD | 2053 | 3619 | 1212 | 3002 |
| P3G6-10 | Average values | 9253 | 3867 | 45519 | 13020 |
| | SD | 1073 | 324 | 1418 | 809 |
| P3G6-13L | Average values | 9148 | 8580 | 63770 | 15740 |
| | SD | 2855 | 2550 | 3125 | 1688 |
| P3G6-13S | Average values | 10640 | 11876 | 45647 | 17098 |
| | SD | 2318 | 451 | 1325 | 1650 |

**Table 12. Fluorescence intensity ratio results**

| Organs | Salivary gland | Liver | Kidney | Intestinal tract |
|---|---|---|---|---|
| P2G6-13L/P2G6-10 | 0.95 | 2.89 | 0.74 | 0.98 |
| P2G6-13S/P2G6-10 | 0.75 | 4.52 | 0.88 | 1.15 |
| P3G6-13L/P3G6-10 | 0.99 | 2.22 | 1.40 | 1.20 |
| P3G6-13S/P3G6-10 | 1.15 | 3.07 | 1.00 | 1.31 |

### Example 6. In vivo Efficacy Assay of GalNAc-siRNA

SPF grade male, 6-8 week old C57BL/6 mice (Beijing Vital River Laboratory Animal Technology Co., Ltd.) were randomly divided into groups. C57/B16 mice were administered via caudal vein. The mouse livers were taken on the 3rd and 7th days and cryopreserved at -80°C respectively. The levels of PCSK9 mRNA in liver tissue were detected. Blood samples were collected from mice on day 3, day 7, day 14, day 21 and day 30, respectively (0.25 ml blood was collected by retrobulbar exsanguination and sent for examination within 1h after blood collection) for detecting the levels of LDL-c, Tc and TG. All animals showed no signs of death or dying during the experiment. No significant abnormalities were observed in all animals.

### First. Detection of expression level of PCSK9 mRNA in mouse liver

Twenty-eight (28) mice were randomly divided into seven groups, four in each group, and the animals grouping and administration are as shown in Table 13. The mouse liver tissue was cryopreserved, and ground by a full-automatic grinding machine. Total RNA was extracted from the mouse liver tissue by Trizol method, and the purity and concentration of RNA were determined by K5500 method to meet the requirements of qPCR. The mRNA expression level of PCSK9 gene was detected by qRT-PCR Starter Kit (Guangzhou RiboBio Co., Ltd.). In the qPCR experiment, three wells were set up for each sample, and the relative expression of PCSK9 mRNA was calculated by 2-ΔΔ Ct method with 18s RNA as an internal reference. The vehicle group was the control group. The relative expression of PCSK9 mRNA was analyzed by the Excel software.

**Table 13. Animal Grouping Table**

| Sequence No | Groupings | sample for test | Dosing (mg/kg) | Volume (mL/kg) |
|---|---|---|---|---|
| 1. | Vehicle control group | Saline | - | 0.2 |
| 2. | Low dose group C | P3G6-03L | 3 | 0.2 |
| 3. | Medium dose group C | P3G6-03L | 10 | 0.2 |
| 4. | High dose group C | P3G6-03L | 33 | 0.2 |
| 5. | Low dose group D | P3G6-03S | 3 | 0.2 |
| 6. | Medium dose group D | P3G6-03S | 10 | 0.2 |
| 7. | High dose group D | P3G6-03S | 33 | 0.2 |

The results showed (Table 14) that expression levels of PCSK9 mRNA in liver tissues of mice in dosing groups C and D were reduced with a certain dose-dependent compared to the vehicle control group from 3 days to 7 days after dosing. The results showed that the target drugs C and D were effective and stable, and could down-regulate the expressions of PCSK9 mRNA in liver tissues of mice.

**Table 14. QPCR quantification for 3-7 day samples**

| No | Groupings | Relative expression on day 3 | Standard deviation on day 3 | Relative expression on day 7 | Standard deviation on day 7 |
|---|---|---|---|---|---|
| 1 | Vehicle control group | 1.00 | 0.17 | 1.00 | 0.23 |
| 2 | Low dose group C | 0.85 | 0.16 | 0.82 | 0.10 |
| 3 | Medium dose group | 0.72 | 0.12 | 0.70 | 0.19 |
| 4 | High dose group C | 0.53 | 0.11 | 0.51 | 0.12 |
| 5 | Low dose group D | 0.95 | 0.21 | 0.80 | 0.21 |
| 6 | Medium dose group | 0.75 | 0.07 | 0.67 | 0.12 |
| 7 | High dose group D | 0.57 | 0.04 | 0.53 | 0.08 |

### Second. Detection of mouse serum biochemical indicators

One hundred and twelve (112) mice were randomly divided into seven groups, sixteen (16) in each group, and the animals grouping and administration are shown in Table 13. 200 µL of blood was collected and centrifuged at 4000 rpm, the supernatant was taken for detection of the serum biochemical indexes by Mairui BS-490 biochemical analyzer.

The results are shown in Tables 15-19.

**Table 15. Statistical table of blood biochemical indexes for animals administered for 3 days**

| Groupings | LDL-C | TC | TG |
|---|---|---|---|
| | mmol | mmol | mmol |
| Vehicle control group (0 mg/kg) | 0.18±0.01 | 2.54±0.17 | 0.85±0.05 |
| Drug C low dose group (3 mg/kg) | 0.17±0.03 | 2.21±0.30 | 0.64±0.19 |
| Drug C middle dose group (10 mg/kg) | 0.16±0.02 | 2.10±0.13 | 0.53±0.03 |
| Drug C high dose group (33 mg/kg) | 0.15±0.01 | 2.08±0.17 | 0.49±0.07 |
| Drug D low dose group (3 mg/kg) | 0.18±0.06 | 2.47±0.15 | 0.74±0.16 |
| Drug D medium dose group (10 mg/kg) | 0.17±0.02 | 2.33±0.11 | 0.58±0.05 |
| Drug D high dose group (33 mg/kg) | 0.16±0.02 | 2.25±0.09 | 0.52±0.04 |

**Table 16. Statistical table of blood biochemical indexes for animals administered for 7 days**

| Groupings | LDL-C | TC | TG |
|---|---|---|---|
| | mmol | mmol | mmol |
| Vehicle control group (0 mg/kg) | 0.16±0.01 | 2.54±0.23 | 0.84±0.17 |
| Drug C low dose group (3 mg/kg) | 0.14±0.02 | 2.44±0.16 | 0.82±0.18 |
| Drug C middle dose group (10 mg/kg) | 0.14±0.02 | 2.15±0.16 | 0.75±0.21 |
| Drug C high dose group (33 mg/kg) | 0.13±0.02 | 2.04±0.21 | 0.68±0.15 |
| Drug D low dose group (3 mg/kg) | 0.15±0.03 | 2.49±0.23 | 0.84±0.17 |
| Drug D medium dose group (10 mg/kg) | 0.14±0.03 | 2.28±0.10 | 0.78±0.18 |
| Drug D high dose group (33 mg/kg) | 0.13±0.01 | 2.17±0.08 | 0.73±0.12 |

**Table 17. Statistical table of blood biochemical indexes for animals administered for 14 days**

| Groupings | LDL-C | TC | TG |
|---|---|---|---|
| | mmol | mmol | mmol |
| Vehicle control group (0 mg/kg) | 0.25±0.04 | 2.93±0.16 | 1.60±0.36 |
| Drug C Low dose group (3 mg/kg) | 0.22±0.08 | 2. 50±0.16 | 1.44±0.34 |
| Drug C Middle dose group (10 mg/kg) | 0.20±0.03 | 2.48±0.14 | 1.40±0.24 |
| Drug C High dose group (33 mg/kg) | 0.18±0.02 | 2.38±0.09 | 1.09±0.33 |
| Drug D Low dose group (3 mg/kg) | 0.24±0.07 | 2.80±0.15 | 1.57±0.22 |
| Drug D medium dose group (10 mg/kg) | 0.21 ±0.05 | 2.55±0.13 | 1.49±0.19 |
| Drug D High dose group (33 mg/kg) | 0.19±0.02 | 2.44±0.11 | 1.23±0.20 |

**Table 18. Statistical table of blood biochemical indexes for animals administered for 21 days**

| Groupings | LDL-C | TC | TG |
|---|---|---|---|
| | mmol | mmol | mmol |
| Vehicle control group (0 mg/kg) | 0.24±0.02 | 2.78±0.14 | 11.50±0.76 |
| Drug C low dose group (3 mg/kg) | 0.22±0.03 | 2.68±0.10 | 1.15±0.38 |
| Drug C middle Dose Group (10 mg/kg) | 0.21±0.02 | 2.65±0.23 | 1.10±0.36 |
| Drug C high dose group (33 mg/kg) | 0.20±0.03 | 2.49±0.15 | 1.07±0.27 |
| Drug D low dose group (3 mg/kg) | 0.24±0.05 | 2.82±0.13 | 1.27±0.25 |
| Drug D medium dose group (10 mg/kg) | 0.23±0.02 | 2.75±0.16 | 1.19±0.21 |
| Drug D high dose group (33 mg/kg) | 0.21±0.02 | 2.63±0.12 | 1.05±0.13 |

**Table 19. Statistical Table of Blood Biochemical Indexes for Animals Administered for 30 Days**

| Groupings | LDL-C | TC | TG |
|---|---|---|---|
| | mmol | mmol | mmol |
| Vehicle control group (0 mg/kg) | 0.19±0.02 | 2.84±0.20 | 0.72±0.16 |
| Drug C low dose group (3 mg/kg) | 0.18±0.02 | 2.74±0.16 | 0.71±0.22 |
| Drug C middle dose group (10 mg/kg) | 0.16±0.02 | 2.73±0.15 | 0.67±0.17 |
| Drug C high dose group (33 mg/kg) | 0.16±0.03 | 2.65±0.18 | 0.60±0.13 |
| Drug D low dose group (3 mg/kg) | 0.18±0.02 | 2.80±0.021 | 0.70±0.18 |
| Drug D middle dose group (10 mg/kg) | 0.17±0.02 | 2.78±0.12 | 0.68±0.17 |
| Drug D high dose group (33 mg/kg) | 0.16±0.01 | 2.69±0.17 | 0. 63±0.12 |

The results showed that (Tables 15-19), after 3-30 days of administration, the expression of LDL-C, TC, TG in animals of both groups C and D were decreased, and the inhibitory effect on the expression of LDL-C, TC, TG gradually increased with the increasing dose; partial doses (e.g., medium and high dose groups) have a more pronounced lipid lowering effect. Therefore, the siRNA molecules of the present disclosure, which inhibit PCSK9 gene expression, represented by the target drugs C and D, have a significant lipid-lowering effect and can be used for the treatment of diseases or symptoms mediated by PCSK9 genes such as cardiovascular diseases or neoplastic diseases.

Although specific embodiments of the present disclosure have been described in detail, those skilled in the art will appreciate that various modifications and variations of the details are possible in light of the above teachings and are within the purview of this disclosure. The full scope of the present disclosure is indicated by the appended claims and any equivalents thereof.

### References

Hutvagner G,Mclachlan J,Pasquinelli A E,et al.A cellular function for the RNA-interference enzyme Dicer in the maturation of the let-7small temporal RNA[J].Science,2001,293(5531):834-8.

Elbashir S M,Harborth J,Lendeckel W,et al.Duplexes of 21-nucleotide RNAs mediate RNA interference in cultured mammalian cells.[J].Nature,2001,411(24):494-8.

Choi J Y,Borch R F.Highly efficient synthesis of enantiomerically enriched2-hydroxymethylaziridines by enzymatic desymmetrization[J].Cheminform,2010,38(18):215-8.

Zamore P D.RNA interference:listening to the sound of silence[J].Nature Structural Biology,2001,8(9):746-50.

## Claims

1. An siRNA molecule for inhibiting expression of PCSK9 gene comprising a sense strand and an antisense strand complementary to form a double strand, wherein the sense strand and/or the antisense strand comprises or consists of 15-27 nucleotides, and the antisense strand is complementary to at least 15, 16, 17, 18, 19, 20, 21, 22, 23, 24, or 25 contiguous nucleotides of SEQ ID NO:1, and wherein at least one nucleotide in the siRNA molecule is modified.

2. The siRNA molecule of claim 1, wherein the sense strand of the siRNA molecule comprises the nucleotide sequence of SEQ ID NO: 1 or a nucleotide sequence of SEQ ID NO: 2; and the antisense strand of the siRNA molecule comprises a nucleotide sequence of SEQ ID NO: 3.

3. The siRNA molecule of claim 1 or 2, wherein the modification comprises locked nucleic acid (LNA), unlocked nucleic acid (UNA), 2'-methoxyethyl, 2'-O-alkyl, 2'-O-methyl, 2'-O-allyl, 2'-C-allyl, 2'-fluoro, 2'-deoxy, 2'-hydroxy, phosphate backbone, DNA, fluorescent probe, ligand modification or combination thereof, preferably 2'-O-methyl, DNA, 2'-fluoro, thio-modified phosphate backbone or combination thereof.

4. The siRNA molecule of claim 3, wherein the sense and antisense strands are selected from the following combinations: strands 1 and 2; strands 3 and 5; strands 3 and 6; strands 3 and 7; strands 3 and 8; strands 9 and 4; strands 9 and 5; strands 9 and 6; strands 9 and 7; and strands 9 and 8.

5. The siRNA molecule of claim 3, wherein the ligand modification is performed at the 3' end, 5' end or in the middle of the sequence of the siRNA molecule; wherein the ligand moiety is Xm, wherein X is the same or different ligand selected from cholesterol, biotin, vitamins, galactose derivatives or analogs, lactose derivatives or analogs, N-acetylgalactosamine derivatives or analogs, N-acetylglucosamine derivatives or analogs, and any combination thereof, m is the number of ligands, preferably m = any integer from 1 to 5, more preferably m = any integer from 2 to 4, most preferably m is 3.

6. The siRNA molecule of claim 5, wherein X has structure Z: 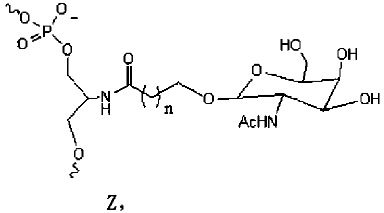 wherein the n value of the CH₂ group in structure Z is independently selected from 1-15; preferably, n in structure Z is 3 or 8; more preferably, when m is 2, 3 or 4, the ligand moiety is (Z)₂, (Z)₃ or (Z)₄, respectively, most preferably each n value in (Z)₂, (Z)₃ or (Z)₄ is equal.

7. The siRNA molecule of claim 4, further comprising a ligand and/or a fluorescent modification, wherein the ligand modification moiety is Xm, wherein X is the same or different ligand selected from cholesterol, biotin, vitamins, galactose derivatives or analogs, lactose derivatives or analogs, N-acetylgalactosamine derivatives or analogs, N-acetylglucosamine derivatives or analogs, and any combination thereof, m is the number of ligands, preferably m = any integer from 1 to 5, more preferably m = any integer from 2 to 4, most preferably m is 3.

8. The siRNA molecule of claim 7, wherein the sense and antisense strands are selected from the following combinations: strands 13 and 8; strands 17 and 8; strands 19 and 8; strands 20 and 8; strands 13 and 10; strands 17 and 10; strands 19 and 10; strands 20 and 10; strands 14 and 10; strands 18 and 10; strands 21 and 10; and strands 22 and 10.

9. The siRNA molecule of claim 7, having structures: A: GACGAUGCCUGCCUCUACU-(X)m; fAmGfUfAfG(s)dA(s)dG(s)dGfCfAfG(s)dG(s)dC(s)dAfUfCmGfUfC(s)mC(s)mC; or B: mGmAmCfGfAfUmGmCmCfUfGfCfCmUfCfUmAmCmU-(X)mf fAmGfUfAfG(s)dA(s)dG(s)dGfCfAfG(s)dG(s)dC(s)dAfUfCmGfUfC(s)mC(s)mC;
wherein X has a structure of Z: 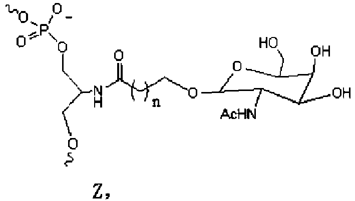 m has a value of 2 or 3 or 4; independently, the n value of the CH₂ group in Z is selected from 1-15; preferably, when m is 2, 3 or 4, the ligand moiety is (Z)₂, (Z)₃ or (Z)4, respectively, and each n value in (Z)₂, (Z)₃ or (Z)₄ is equal.

10. The siRNA molecule of claim 9 having an n value of 3 or 8.

11. The siRNA molecule of any one of claims 1-10, for inhibiting the expression of the PCSK9 gene in humans and monkeys.

12. A pharmaceutical composition comprising an siRNA molecule of any one of claims 1-10 and pharmaceutically acceptable other components.

13. A method for inhibiting or reducing the expression level of the PCSK9 gene in a cell in vivo or in vitro, comprising introducing into the cell the siRNA molecule of any one of claims 1-10, or the pharmaceutical composition of claim 12, such that the expression level of the PCSK9 gene is inhibited or reduced by at least 95%, at least 90%, at least 85%, at least 80%, at least 75%, at least 70%, at least 60%, at least 50%, at least 40%, at least 30%, at least 20%, at least 10%, or at least 5%.

14. Use of the siRNA molecule of any one of Claims 1-10 or the pharmaceutical composition of Claim 12 for the manufacture of a medicament for inhibiting or reducing the expression level of the PCSK9 gene in a cell in vivo or in vitro or for the manufacture of a medicament for treating diseases or symptoms mediated by the PCSK9 gene in a subject.

15. The use of claim 14, wherein the diseases or symptoms mediated by the PCSK9 gene comprises a cardiovascular disease or a neoplastic disease, preferably the cardiovascular disease is selected from hyperlipidemia, hypercholesterolemia, non-familial hypercholesterolemia, polygenic hypercholesterolemia, familial hypercholesterolemia, homozygous familial hypercholesterolemia, or heterozygous familial hypercholesterolemia and the neoplastic disease is selected from melanoma, hepatocellular carcinoma, and metastatic liver cancer.

16. A kit comprising an siRNA molecule of any one of claims 1-11.

17. A method for treating diseases or symptoms mediated by the PCSK9 gene in a subject, comprising administering to the subject the siRNA molecule of any one of Claims 1-11 or the pharmaceutical composition of Claim 12.

18. The method of claim 17, wherein the diseases or symptoms mediated by the PCSK9 gene comprises a cardiovascular disease or a neoplastic disease, preferably the cardiovascular disease is selected from hyperlipidemia, hypercholesterolemia, non-familial hypercholesterolemia, polygenic hypercholesterolemia, familial hypercholesterolemia, homozygous familial hypercholesterolemia, or heterozygous familial hypercholesterolemia and the neoplastic disease is selected from melanoma, hepatocellular carcinoma, and metastatic liver cancer.

19. Use of an siRNA molecule of any one of claims 1-11 or a pharmaceutical composition of claim 12 in the manufacture of a medicament for inhibiting or reducing the level of expression of the PCSK9 gene in a cell in vivo or in vitro.

20. Use of an siRNA molecule of any one of claims 1-11 or the pharmaceutical composition of claim 12 for inhibiting or reducing the expression level of the PCSK9 gene in cells in vivo or in vitro.
